# EUROPEAN PATENT APPLICATION

(11) **EP 2 481 552 A1**
(43) Date of publication of application: **01.08.2012**
(21) Application number: 12152102.5
(22) Date of filing: 23.01.2012
(51) Int. Cl.: B29C 47/26, B29C 47/06, A61M 25/00, B29C 47/92

(54) **Extrusion molding die, extruding molding apparatus, medical tube, and method for producing medical tube**

(30) Priority: 26.01.2011 JP 2011013996
(71) Applicant: Tyco Healthcare Group LP, Mansfield, MA 02048 (US)
(72) Inventor: Ohigawa, Atsushi, Tokyo 158-8615 (JP); Tamura, Tomoyuki, Tokyo 158-8615 (JP); Yokota, shinichiro, Tokyo 158-8615 (JP)
(74) Representative: Gray, James

(57) **Abstract**

An extrusion molding die (50) is provided which produces a tube that contains a portion in which the cross-sectional area ratio of an inner layer and outer layer in axial cross-section continuously varies, wherein said cross-sectional area ratio can be changed in a short time. The extrusion molding die (50) moves a second inner layer mandrel (54) in the axial direction and changes the flow passage volume of an inner layer resin flow passage (62a) in accordance with changes in supply quantity of an inner layer forming resin.

## Description

### Technical Field

The present invention relates to an extrusion molding die used when producing a tube that contains a portion in which the cross-sectional area ratio of the inner layer and outer layer in the axial cross-section continuously varies, an extrusion molding apparatus which uses the extrusion molding die, a medical tube produced using the extrusion molding apparatus, and a method for producing the medical tube.

### Background Art

Since the past, medical tubes have been widely use in the field of medicine as catheters and so forth. In a medical tube used while inserted inside the body like a catheter, its tip end must reach the prescribed site without injuring the site in the body where it is inserted. That is, in such a medical tube, ease of insertion into the body and suppression of injury inside the body are required. Additionally, in a medical tube used while inserted inside the body like a catheter, it is also required that insertion force and torque are reliably transmitted from the base end to the tip end. To satisfy these requirements, medical tubes are generally molded such that the tip end is relatively soft and the main body which includes the base end is relatively hard.

Medical tubes are generally produced by extrusion molding so that the tip end does not fall off and so that no steps or gaps, etc., are produced at the interface between the tip end and the main body. Using extrusion molding, a medical tube is molded such that the cross-sectional area ratio of the inner layer and outer layer in the axial cross-section of the medical tube changes, and such that the tip end is relatively soft and the main body which includes the base end is relatively hard, with a hard-soft transition part interposed. In general, an extrusion device which supplies resin and whose main part is a screw, etc., is attached to such an extrusion molding die. Additionally, various extrusion molding dies in which the cross-sectional area ratio of the inner layer and outer layer in the axial cross-section is changed by using a multilayer die and adjusting the amount of resin supplied from the extrusion device have been proposed.

For example, there is one that can adjust the amount of supplied resin by controlling the degree of openness of a valve (for example, an electromagnetic valve, etc.) provided in the die, and one that can adjust the amount of supplied resin by variably controlling the rotational speed of the screw. Such techniques are disclosed as "two or more polymers having different moduli of elasticity are continuously alternately supplied to a long land die in which a molding aid has been supplied to the inner surface, and they are extruded by molding and cooling in said long land die" (for example, refer to Japanese Unexamined Patent Application Publication No. H05-49698, Patent Reference 1).

Also, a technique is disclosed as "an extrusion-molded body obtained by continuously extrusion molding at least two types of first resin and second resin, which is made up of a part made of only the first resin, a part made of only the second resin, and a transition part between them containing both resins, wherein, in said transition part, the two resins have been mixed in a nearly homogeneous state, and their blending ratio gradually changes such that the proportion of the second resin increases from the first resin along the direction of extrusion" (for example, refer to Japanese Unexamined Patent Application Publication No. 2001-293770, Patent Reference 2).

Incidentally, resin flow passages in which the resins flow are formed in the extrusion molding die. Because the resin flow passages have a prescribed length, the pressure applied to the resin inside the resin flow passages ends up remaining as residual pressure even when the extrusion device stops. A technique for reducing this residual pressure is disclosed as "an extrusion device comprising a conical nipple and a die separated by a fixed gap surrounding this nipple inside a crosshead into which molten resin is supplied, in which said molten resin flows from this gap to the tip side of said nipple and is extruded on and made to cover a core wire drawn continuously from the tip end of the nipple, wherein a ring-shaped protrusion is formed surrounding said nipple or on the inner circumferential surface of said die, and said die or nipple is provided such that they can move toward and away from each other" (refer to Japanese Unexamined Patent Application Publication No. 2000-326384, Patent Reference 3).

The one that can adjust the cross-sectional area ratio of the inner layer and outer layer in the axial cross-section by controlling the degree of openness of a valve provided in the die is applicable to extrusion molding in which there is a core in the inner cavity of a catheter for vascular contrast or a guide wire, etc., but since a sudden change in pressure occurs the moment the valve is switched, it is not applicable to extrusion molding in which there is no core in the inner cavity (plain drawing extrusion molding). Also, the one that can change the cross-sectional area ratio of the inner layer and outer layer in the axial cross-section by controlling the rotational speed of the screw is applicable to extrusion molding in which there is no core in the inner cavity, but since the volume of resin in the die becomes large, the pressure applied to the resin becomes residual pressure, and in a narrow tube such as a medical tube, the hard-soft transition part ends up being long.

In both the one that can adjust the amount of supplied resin by controlling the degree of openness of a valve provided in the die and the one that can adjust the amount of supplied resin by variably controlling the rotational speed of the screw, if a short hard-soft transition part is desired, the hard-soft resin merge part can be brought closer to the tip side. However, if the hard-soft resin merge part is simply brought closer to the tip side, the dimensions of the tube end up becoming unstable. In this case, a tube having dimensions within the prescribed range cannot be produced, and yield is extremely low.

In the technique according to Patent Reference 1, a long land die is provided in order to stabilize molding. However, since the structure of a long land die is extremely complex, it is not applicable to extrusion molding of tubes having a heteromorphous cross-section. The technique according to Patent Reference 2 can adjust the amount of supplied resin by controlling a screw or valve. However, since a core wire is used in molding of the tube, it is not applicable to extrusion molding of tubes having a heteromorphous cross-section. Further, the techniques according to Patent Reference 1 and Patent Reference 2 make no mention at all of residual pressure inside the resin flow passages. Additionally, the technique according to Patent Reference 2 is not applicable to cases where different characteristics of resins are desired in the inner layer and outer layer (for example, hardness or color, chemical resistance), because extrusion is performed in a state where the two or more types of resins are mixed nearly homogenously in the transition part.

Patent Reference 3 states that "using this, different materials can be continuously coated in the long direction, and therefore it can exhibit the superior effect that a high-quality tube constructed of resins of different hardness in the long direction, as in a catheter, etc., can be easily produced" (paragraph [0034] of Patent Reference 3). However, Patent Reference 3 discloses a technique in which the boundary between the coated and uncoated parts is made clear, and does not allude to the resin proportion varying continuously. Moreover, since the technique according to Patent Reference 3 relates to intermittent extrusion molding, it cannot be affirmed as a combination of Patent Reference 1 and Patent Reference 2. In addition, because the technique according to Patent Reference 3 uses a core wire in tube molding, it is not applicable to extrusion molding of tubes having a heteromorphous cross-section.

### Summary of the Invention

The present invention was devised to solve the above problems, and its objective is to provide an extrusion molding die used when producing a tube that contains a portion in which the cross-sectional area ratio of the inner layer and outer layer in the axial cross-section continuously varies, an extrusion molding apparatus which uses the extrusion molding die, a medical tube produced using the extrusion molding apparatus, and a method for producing the medical tube.

The extrusion molding die according to the present invention is an extrusion molding die which extrusion-molds a tube containing a portion in which at least an inner layer and an outer layer are formed by resin, in which the cross-sectional area ratio of the inner layer and outer layer in the axial cross-section of the portion varies continuously, comprising a main body; a first inner layer mandrel affixed to the main body, on the tip end of which a land part is formed; a second inner layer mandrel arranged such that the inner circumferential surface abuts the outer circumferential surface of the first inner layer mandrel, which can move in the axial direction; an outer layer mandrel having a first through-hole through which the first inner layer mandrel and the second inner layer mandrel are inserted; and a body having a second through-hole through which the outer layer mandrel is inserted; wherein an outer layer resin flow passage, through which outer layer forming resin which forms the outer layer flows, is constructed from the outer circumferential surface of the outer layer mandrel and the inner circumferential surface of the second through-hole; an inner layer resin flow passage, through which inner layer forming resin which forms the inner layer flows, is constructed from a part of the outer circumferential surface of the second inner layer mandrel and a part of the outer circumferential surface of the first inner layer mandrel and the inner circumferential surface of the first through-hole; a merged resin flow passage, in which the outer layer resin flow passage and the inner layer resin flow passage merge, is constructed from the outer circumferential surface of the tip side that contains the land part of the first inner layer mandrel and the inner circumferential surface of the second through-hole; and the second inner layer mandrel is moved in the axial direction and the flow passage volume of the inner layer resin flow passage is changed in accordance with changes in the supply quantity of the outer layer forming resin and/or the inner layer forming resin.

In the extrusion molding die according to the present invention, a merge part adjusting collar which enables adjustment of the distance from the merge part, in which the outer layer resin flow passage and the inner layer resin flow passage merge, to the tip end of the land part is provided between the body and the outer layer mandrel.

The extrusion molding apparatus according to the present invention is an extrusion molding apparatus comprising the aforementioned extrusion molding die, and a drive unit which drives the second inner layer mandrel.

In the extrusion molding apparatus according to the present invention, a controller which controls driving of the second inner layer mandrel is provided, and the controller controls driving of the second inner layer mandrel so as to enlarge the flow passage volume of the inner layer resin flow passage when the supply quantity of the outer layer forming resin and/or the inner layer forming resin is decreased.

The medical tube according to the present invention is a medical tube extrusion-molded by the aforementioned extrusion molding apparatus, which has a transition part which contains a portion in which the cross-sectional area ratio of resin of the inner layer and outer layer in the axial cross-section continuously varies.

The method for producing a medical tube according to the present invention is a method for producing a medical tube which contains a portion in which at least an inner layer and outer layer are formed by resin, in which the cross-sectional area ratio of resin of the inner layer and outer layer in the axial cross-section of the portion continuously varies, wherein, when the supply quantity of the outer layer forming resin and/or the inner layer forming resin is decreased from the current supply quantity, residual pressure in the inner layer resin flow passage, in the outer layer resin flow passage through which the outer layer resin flows, and in the merged resin flow passage through which merged resin of the inner layer forming resin and the outer layer forming resin flows is decreased by enlarging the flow passage volume of the inner layer resin flow passage through which the inner layer forming resin flows.

### Advantageous Effects of the Invention

According to the extrusion molding die according to the present invention, a second inner layer mandrel is moved in the axial direction and the flow passage volume of the inner layer resin flow passage is changed in accordance with changes in the supply quantity of the outer layer forming resin and/or the inner layer forming resin. As a result, the pressure inside the resin flow passages (inner layer resin flow passage, outer layer resin flow passage, merged resin flow passage) can be controlled, and the cross-sectional area ratio of the inner layer and outer layer in the axial cross-section can be changed in a short time. Further, according to the extrusion molding die according to the present invention, the length of the transition part in which the cross-sectional area ratio of the inner layer and outer layer in the axial cross-section continuously varies while dimensional stability is maintained can be selected in accordance with the application of the tube.

According to the extrusion molding die according to the present invention, a merge part adjusting collar which enables adjustment of the distance from the merge part, in which the outer layer resin flow passage and inner layer resin flow passage merge, to the tip end of the land part is provided between the body and the outer layer mandrel. As a result, the dimensional stability of the tube can be additionally maintained.

According to the extrusion molding apparatus according to the present invention, the aforementioned extrusion molding die and a drive part that drives the second inner layer mandrel are provided. As a result, the second inner layer mandrel can be moved in the axial direction without a complex configuration.

According to the extrusion molding apparatus according the present invention, a controller drives the second inner layer mandrel so as to enlarge the flow passage volume of the inner layer resin flow passage when the supply quantity of the outer layer forming resin and/or the inner layer forming resin is decreased. As a result, when the supply quantity of the outer layer forming resin and/or the inner layer forming resin is decreased, residual pressure in the inner layer resin flow passage, in the outer layer resin flow passage through which the outer layer resin flows, and in the merged resin flow passage through which merged resin of the inner layer forming resin and the outer layer forming resin flows is decreased by enlarging the flow passage volume of the inner layer resin flow passage through which the inner layer forming resin flows.

According to the medical tube according to the present invention, it is extrusion-molded by the aforementioned extrusion molding apparatus, and has a transition part which contains a portion in which the cross-sectional area ratio of resin of the inner layer and outer layer in the axial cross-section continuously varies. As a result, it can be used in a variety of applications.

According to the medical tube according to the present invention, when the supply quantity of the outer layer forming resin and/or the inner layer forming resin is decreased from the current supply quantity, residual pressure in the inner layer resin flow passage, in the outer layer resin flow passage through which the outer layer resin flows, and in the merged resin flow passage through which merged resin of the inner layer forming resin and the outer layer forming resin flows is decreased by enlarging the flow passage volume of the inner layer resin flow passage through which the inner layer forming resin flows. As a result, the cross-sectional area ratio of the inner layer and outer layer in the axial cross-section can be changed in a short time.

### Brief Description of the Drawings

FIG. 1 is a schematic cross-sectional view illustrating the cross-sectional structure of the extrusion molding die according to embodiment 1 of the present invention;
FIG. 2 is a schematic cross-sectional view illustrating the cross-sectional structure of the extrusion molding die according to embodiment 1 of the present invention;
FIG. 3 is an enlarged schematic cross-sectional view illustrating an enlarged cross-section of the main parts of the extrusion molding die according to embodiment 1 of the present invention;
FIG. 4 is an enlarged schematic cross-sectional view illustrating an enlarged cross-section of the main parts of the extrusion molding die according to embodiment 1 of the present invention;
FIG. 5 is a schematic view illustrating the extrusion molding die according to embodiment 1 of the present invention in the state seen from the front side;
FIG. 6 is a schematic cross-sectional view illustrating the cross-sectional structure of the extrusion molding die according to embodiment 2 of the present invention;
FIG. 7 is a schematic cross-sectional view illustrating the cross-sectional structure of the extrusion molding die according to embodiment 2 of the present invention;
FIG. 8 is an enlarged schematic cross-sectional view illustrating an enlarged cross-section of the main parts of the extrusion molding die according to embodiment 2 of the present invention;
FIG. 9 is an enlarged schematic cross-sectional view illustrating an enlarged cross-section of the main parts of the extrusion molding die according to embodiment 2 of the present invention;
FIG. 10 is a schematic view illustrating the cross-sectional structure of the extrusion molding apparatus according to embodiment 3 of the present invention.;
FIGS. 11(a)-(d) are explanatory diagrams for explaining the medical tube according to embodiment 4 of the present invention;
FIGS. 12(a)-(d) are explanatory diagrams for explaining the medical tube of another configuration according to embodiment 4 of the present invention;
FIG. 13 is an explanatory diagram for explaining the method for producing a medical tube according to embodiment 5 of the present invention; and
FIG. 14 is a timing chart illustrating the operation timing of each device in the method for producing a medical tube according to embodiment 5 of the present invention.

### Detailed Description of Embodiments

Embodiments of the present invention will be described below based on the drawings.

### Embodiment 1

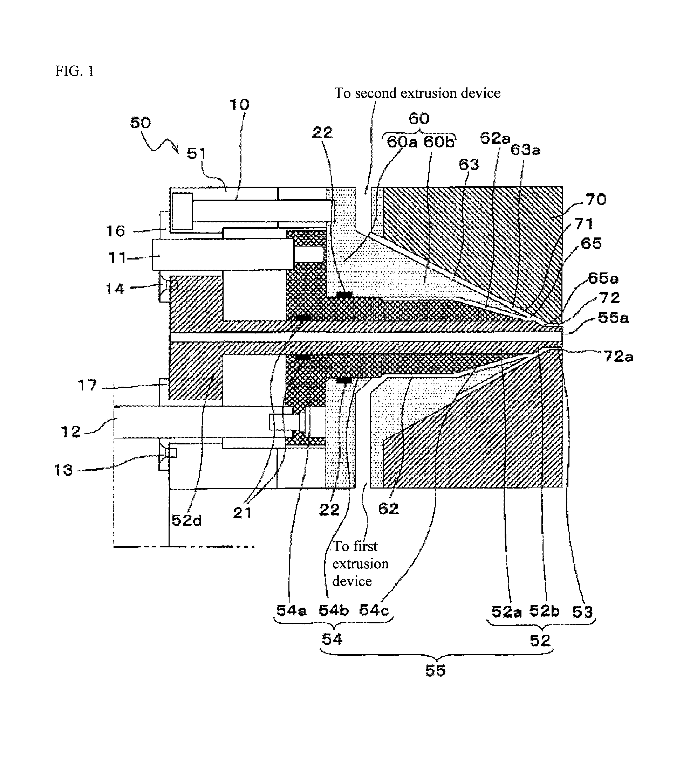

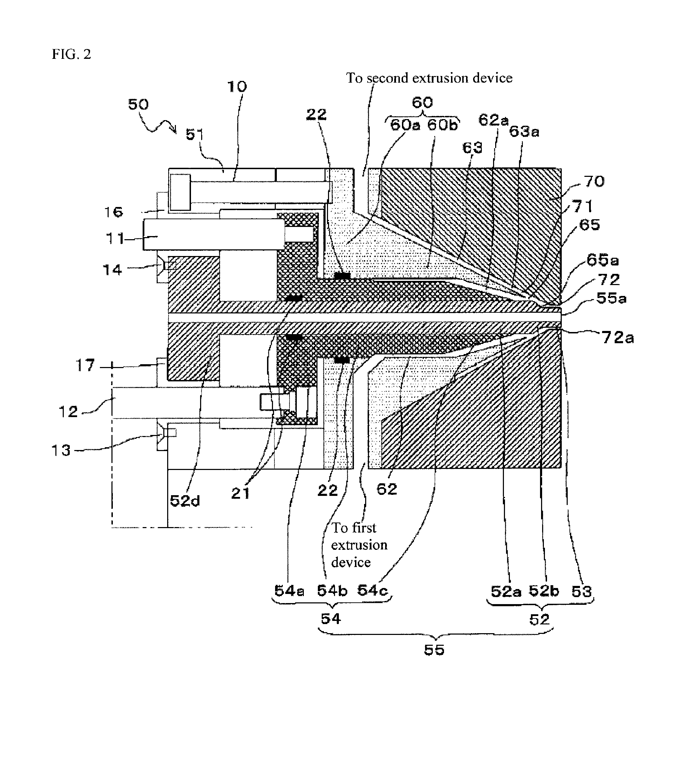

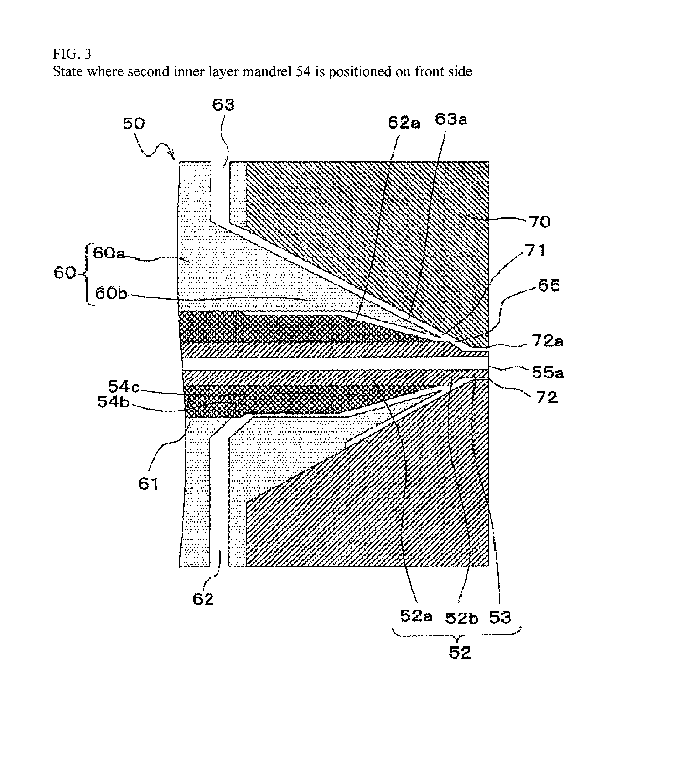

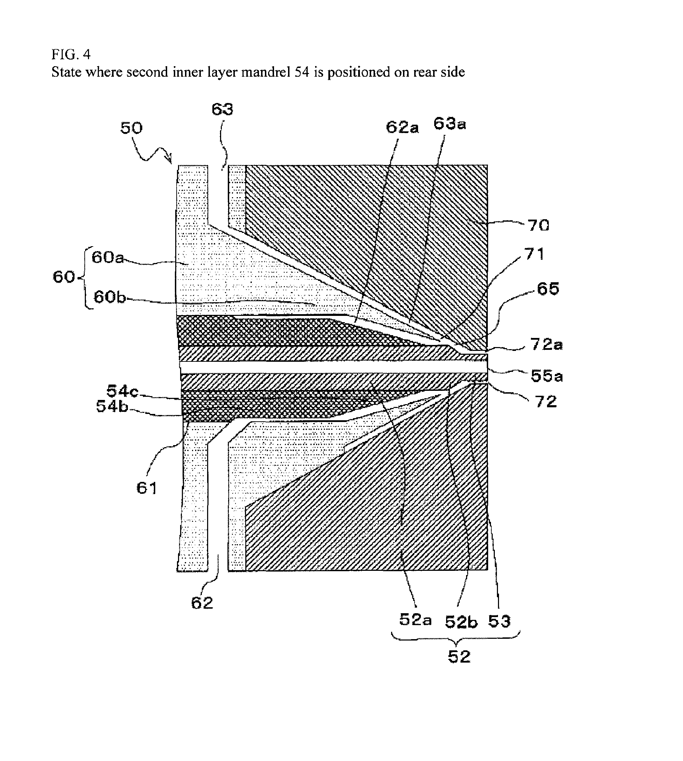

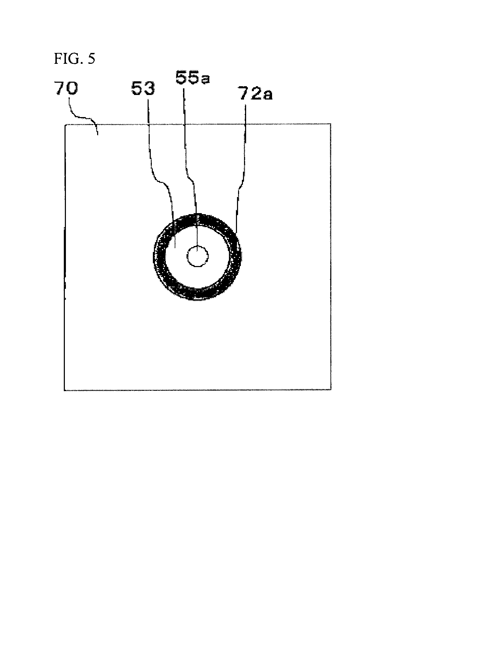

FIGS. 1 and 2 are schematic cross-sectional views illustrating the cross-sectional structure of an extrusion molding die 50 according to embodiment 1 of the present invention. FIGS. 3 and 4 are enlarged schematic cross-sectional views illustrating enlarged cross-sections of the main parts of the extrusion molding die 50. FIG. 5 is a schematic view illustrating the extrusion molding die 50 in the state seen from the front side. The configuration and operation of the extrusion molding die 50 will be described based on FIGS. 1-5. FIGS. 1 and 3 show the state where the second inner layer mandrel 54 is positioned on the front side, and FIGS. 2 and 4 show the state where the second inner layer mandrel 54 is positioned on the rear side. Note that there are cases where the relationships among the sizes of the constituent components differ in the drawings below, including FIG. 1. Also, the front side refers to the land part side (the side right of the central axis in the drawings), and the rear side refers to the side opposite the land part side (the side left of the central axis in the drawings).

The extrusion molding die 50 according to embodiment 1 of the present invention continuously supplies the same resin or a plurality of types of resin via at least two resin flow passages, and is used when producing a tube having at least an inner layer and an outer layer (for example, a medical tube). Also, the tube molded by the extrusion molding die 50 has a transition part in which the properties smoothly change (for example, a hard-soft transition part or color transition part), which contains a portion in which the cross-sectional area ratio of resin of the inner layer and outer layer in the axial cross-section continuously varies. That is, the tube molded in the extrusion molding die 50 can exhibit the different properties (for example, hardness or color, chemical resistance, contrast capability, elasticity, body tissue compatibility, surface smoothness, etc.) of the resins of the inner layer and outer layer. Also, such characteristics may be combined as appropriate. For example, in the case where a medical tube is molded, the inside layer is normally formed of resin having chemical resistance, and therefore, the medical tube is made such that the supply quantity of resin that forms the outer layer (for example, a resin which softens at body temperature, etc.) can be increased or decreased while maintaining the characteristics of the inner layer.

To cite a specific example, the inner layer may be formed by a resin material having a primary component of chemical-resistant aromatic polyurethane, and the outer layer may be formed by a resin material having a primary component of aliphatic polyurethane which softens at body temperature, and a medical tube may be molded by increasing and decreasing the supply quantities thereof. The resin material having a primary component of aromatic polyurethane has the characteristic of not degrading even when in contact with anticancer agents, antiseptic agents, etc., for a long period. The resin material having a primary component of aliphatic polyurethane has the characteristics of excellent blendability with X-ray impermeable materials and softening after residing in the body. If aliphatic polyurethane is used, a contrasting part can be formed by blending an X-ray impermeable material in the aliphatic polyurethane. Also, operability during insertion is greatly improved and compatibility with the body is excellent compared to the case where the entire medical tube is constructed of aromatic polyurethane.

The extrusion molding die 50 is schematically constructed of a main body 51 which holds the components, a first inner layer mandrel 52 affixed inside the body 51, a second inner layer mandrel 54 arranged inside the main body 51 such that its inner circumferential surface abuts the outer circumferential surface of the first inner layer mandrel 52, an outer layer mandrel 60 arranged with a prescribed gap (inner layer resin flow passage 62a described below) from the outer circumferential surface of the second inner layer mandrel 54, and a body 70 which holds the outer layer mandrel 60, arranged with a prescribed gap (outer layer resin flow passage 63a described below) from the outer layer mandrel 60. Note that the tip end of the first inner layer mandrel 52 is configured as a land part 53.

When a tube is formed, the outer layer mandrel 60 serves the function of guiding the flow of the outer layer forming resin which forms the outer layer of the tube. The outer layer mandrel 60 comprises a pedestal 60a affixed to the main body 51, and a tip side tapered part 60b arranged in a tapered hole 71 formed in the body 70 with the outer layer resin flow passage 63a interposed. Also, inside the outer layer mandrel 60, an outer layer mandrel through-hole (first through-hole) 61 which passes through it in the axial direction is formed. The first inner layer mandrel 52 and the second inner layer mandrel 53 are arranged in this outer layer mandrel through-hole 61.

Furthermore, in the outer layer mandrel 60, a first resin flow passage 62 and a second resin flow passage 63 are formed so as to connect through from the outer circumferential surface to the outer layer mandrel through-hole 61. The outer layer mandrel through-hole 61 is constructed of a circumferential surface which tapers off as it runs from the inlet of the first resin flow passage 62 toward the front side. Also, the resin material supplied from the first resin flow passage 62 (called "inner layer forming resin" hereinafter) flows through the inner layer resin flow passage 62, and the resin material supplied from the second resin flow passage 63 (called "outer layer forming resin" hereinafter) flows through the outer layer resin flow passage 63a, and they merge at a merge part 65 between the tip end of the second inner layer mandrel 54 and the land part 53.

The first resin flow passage 62 is formed so as to pass through the inside of the outer layer mandrel 60 and reach the outer layer mandrel through-hole 61. In FIGS. 1-4, the first resin flow passage 62 is formed so as to pass through to a position that abuts the outer circumferential surface of the second inner layer mandrel 54 - that is, to penetrate up to a position that connects through to the outer layer mandrel through-hole 61. From the position where it abuts the outer circumferential surface of the second inner layer mandrel 54 up to the merge part 65, it is formed as a gap (inner layer resin flow passage 62a) between the inner circumferential surface of the outer layer mandrel through-hole 61 and the outer circumferential surface of the first inner layer mandrel 52 and second inner mandrel 54. Also, a first extrusion device (not shown in drawings), having as its primary part a screw whose rotation can be controlled which supplies the inner layer forming resin, is connected to the first resin flow passage 62.

The second resin flow passage 63 is formed so as to reach from the outer circumferential surface of the outer layer mandrel 60 to the merge part 65. In FIGS. 1-4, the second resin flow passage 63 is formed so as to penetrate up to the position where it abuts the outer circumferential surface of the outer layer mandrel 60. From the position where it abuts the outer circumferential surface of the outer layer mandrel 60 up to the merge part 65, it is formed as a gap (outer layer resin flow passage 63a) between the inner circumferential surface of the tapered hole 71 of the body 70 and the outer circumferential surface of the outer layer mandrel 60. Also, a second extrusion device (not shown in drawings), having as its primary part a screw whose rotation can be controlled which supplies the outer layer forming resin, is connected to the second resin flow passage 63.

By means of the first extrusion device and second extrusion device, the supply quantities of inner layer forming resin and outer layer forming resin are adjusted, and the inner layer forming resin is supplied from the first resin flow passage 62 to the inner layer resin flow passage 62a, and the outer layer forming resin is supplied from the second resin flow passage 63 to the outer layer resin flow passage 63a. Also, the inner layer forming resin and outer layer forming resin merge at the merge part 65, such that they can be extruded from the front part in a state where the inner and outer layers have been formed.

The body 70 holds the outer layer mandrel 60 and also forms the outer layer resin flow passage 63a together with the outer layer mandrel 60. Inside the body 70 are provided the tapered hole 71, into which are inserted the tip side of the first inner layer mandrel 52 up to where it reaches the land part 53, and the tip side of the second inner layer mandrel 54, and the tip side tapered part 60b of the outer layer mandrel 60; and a body through-hole (second through-hole) 72 which connects through to the tapered hole 71 and into which the land part 53 of the first inner layer mandrel 52 is inserted. Note that the resin flow passage downstream from the merge part 65 - that is, the resin flow passage formed by the inner circumferential surface of the tapered hole 71, the outer circumferential surface of the tip side reaching up to the land part 53 of the first inner layer mandrel 52 (tapered part 52b described below), and the outer circumferential surface of the land part 53 and the inner circumferential surface of the body through-hole 72 - is called the merged resin flow passage 65a, through which the merged resin flows.

The tapered hole 71 is formed in a substantially conical shape whose back side is large in diameter and front side is small in diameter, in accordance with the shape of the tip side reaching up to the land part 53 of the first inner layer mandrel 52, the tip side of the second inner layer mandrel 54, and the tip side tapered part 60b of the outer layer mandrel 60. The body through-hole 72 is formed in a substantially cylindrical shape which maintains a prescribed diameter, in accordance with the shape of the land part 53. The body through-hole 72 opens into the body 70 via an opening 72a. Also, the merged resin that has flowed through the merged resin flow passage 65a is extruded from between the outer circumferential surface of the land part 53 and the inner circumferential surface of the body through-hole 72 - that is, from the opening 72a - to outside the body 70 (refer to black shaded portion shown in FIG. 5).

Note that the body through-hole 72 formed in the body 70 and the outer layer mandrel through-hole 61 formed in the outer layer mandrel 60 become connected to each other in the state where the outer layer mandrel 60 has been arranged in the body 70. Also, in the descriptions below, there are cases where the body through-hole 72 formed in the body 70 and the outer layer mandrel through-hole 61 formed in the outer layer mandrel 60 are together called the extrusion hole 80.

The first inner layer mandrel 52 is inserted into the outer layer mandrel through-hole 61 of the outer layer mandrel 60, and is constructed of a pedestal 52d affixed to the main body 51 on the rear side, a main body (first inner layer mandrel main body) 52a extending from the pedestal 52d to the front side in a cylindrical shape, a substantially conical tapered part 52b which gradually narrows in diameter in the forward direction from the front end of the main body 52a, and a land part 53 which is formed from the front end of the tapered part 52b to the tip end. Also, an internal pressure adjusting hole 55a is formed so as to pass through inside the first inner layer mandrel 52 in the axial direction. Note that in the drawings, the pedestal 52d is illustrated in a position separated from the other components.

The space between the outer circumferential surface on the side nearest the tapered part 52b of the main body 52a and the inner circumferential surface of the extrusion hole 80 (particularly the outer layer mandrel through-hole 61) constitutes a part of the inner layer resin flow passage 62a, and the space between the outer circumferential surface of the tapered part 52b and the inner surface of the extrusion hole 80 (particularly the body through-hole 72) constitutes the merged resin flow passage 65a through which the merged resin merged at the merge part 65 moves.

The land part 53 provided on the tip end of the tapered part 52b has a prescribed diameter and length in order to stabilize the dimensions of the molded tube. Also, the tip end surface of the land part 53 is positioned on substantially the same plane as the opening surface of the opening 72a of the body through-hole 72 formed in the body 70. Additionally, in the extrusion molding die 50, in order to shorten the transition part which contains a portion in which the cross-sectional area ratio of resin of the inner layer and outer layer in the axial cross-section continuously varies, the volume of the merged resin flow passage 65a is reduced by shortening the distance (length) of the merged resin flow passage 65a - that is, the length in the axial direction of the land part 53 (described below). Note that the internal pressure adjusting hole 55a adjusts the internal pressure of the tube by a fluid such as air when the tube is molded.

The second inner layer mandrel 54 serves the function of guiding the flow of inner layer forming resin which forms the inner layer of the tube when the tube is formed. That is, together with the outer layer mandrel 60, it has the function of forming the inner layer resin flow passage 62a (ring-shaped space formed between the outer layer mandrel through-hole 61 of the outer layer mandrel 60 and the outer circumferential surface of the inner layer mandrel 54) when the second inner layer mandrel 54 is inserted in the outer layer mandrel through-hole 61 of the outer layer mandrel 60.

The second inner layer mandrel 54 is located on the rear side of the pedestal 60a of the outer layer mandrel 60, and is constructed of a support part 54a arranged in an internal space formed in the main body 51, a main body (second inner layer mandrel main body) 54b inserted into the outer layer mandrel through-hole 61 so as to abut the outer circumferential surface of the first inner layer mandrel 52 and the inner circumferential surface of the outer layer mandrel through-hole 61 of the outer layer mandrel 60, and a tip end tapered part 54c having a substantially conical shape which gradually narrows in diameter in the forward direction from the front end of the main body 54b. Note that the inner layer mandrel 55 is constructed of the first inner layer mandrel 52 and the second inner layer mandrel 54. That is, the inner layer mandrel 55 has a two-part structure.

This second inner layer mandrel 54 can move in the axial direction via the support part 54a. That is, by moving the second inner layer mandrel 54 in the axial direction, the flow passage volume of the inner layer resin flow passage 62a can be varied (enlarged, shrunken). As shown in FIG. 2 and FIG. 4, when the second inner layer mandrel 54 is positioned on the rear side, the flow passage volume of the inner layer resin flow passage 62a is enlarged. In this case, it is seen that a gap is formed in the internal space where the support part 54a is arranged (refer to FIG. 2). However, since the first inner layer mandrel 52 is fixed even when the second inner layer mandrel 54 moves, the first inner layer mandrel 52 does not move closer to the inner layer mandrel, and the land part 53 does not protrude on the outer circumferential surface side of the body 70, and the dimensional stability of the molded tube is not affected.

Note that the tip end of the second extrusion device connected to the second resin flow passage 63 may be affixed to the main body 51 by a fixing component 10 in combination with the outer layer mandrel 60. In the state where the second inner layer mandrel 54 is mounted in the extrusion hole 80, the support 54a is connected to a guide 11 and a drive shaft 12. The guide 11 moves in the axial direction together with the second inner layer mandrel 54, and guides the axial-direction movement of the second inner layer mandrel 54. The drive shaft 12 is connected on the other end to the mandrel drive part (not shown in the drawings), and it transmits the driving force of the mandrel drive part to the second inner layer mandrel 54. A support part 16 supports the outer layer mandrel 60 and the inner layer mandrel 55 on the main body 51 via a fixing component 14. A support part 17 supports the outer layer mandrel 60 and the inner layer mandrel 55 on the main body 51 via a fixing component 13.

Furthermore, a seal material 21 for preventing leakage of the resin material is arranged on part of the outer circumferential surface of the main body 52a. An O-ring, etc., conforming to the outer circumferential surface of the main body 52a may be used as this seal material 21. Additionally, a seal material 22 for preventing leakage of the resin material is arranged on part of the outer circumferential surface of the main body 54b. An O-ring, etc., conforming to the outer circumferential surface of the main body 54b may be used as this seal material 22. However, the fixing component 10, guide 11, drive shaft 12, fixing component 13, fixing component 14, support part 16, support part 17, seal material 21 and seal material 22 are not limited to those illustrated in the drawings.

Here, the characteristic features of the extrusion molding die 50 will be described. The extrusion molding die 50 makes it possible to shorten the transition part of the resin materials that constitute the inner and outer layers of the tube as much as possible, to conform to various requirements for tubes. To do so, first, the merged resin flow passage 65a, and in particular the distance in the axial direction of the land part 53, is shortened. However, if the distance of the merged resin flow passage 65a is simply shortened, the tube ends up lacking dimensional stability. Thus, secondly, the second inner layer mandrel 54 is enabled to move in the axial direction. By so doing, the flow passage volume of the inner layer resin flow passage 62a can be enlarged and shrunken, and as a result, residual pressure in the outer layer resin flow passage 62a, outer layer resin flow passage 63a and merged resin flow passage 65a can be reduced. Therefore, according to the extrusion molding die 50, the transition part can be shortened while maintaining dimensional stability. Note that although the reduction in residual pressure is described in detail below, reducing residual pressure also includes the case where residual pressure ends up being released so that it goes to zero.

The operation of the extrusion molding die 50 configured as above will now be described.

When a tube is molded using the extrusion molding die 50, the extrusion molding die 50 is heated to an appropriate temperature. After that, the first resin flow passage 62 and second resin flow passage 63 are filled with their respective resin materials (for example, polyurethane, etc.) in the heated state. Further, the resin materials are fed from the first resin flow passage 62 and second resin flow passage 63, and are moved toward the land part 53. At this time, when the resin materials pass through the inner layer resin flow passage 62a and outer layer resin flow passage 63a, they are formed into a cylindrical shape, and furthermore, they are deformed into a cylindrical shape which gradually decreases in diameter while it passes through the merged resin flow passage 65a.

Specifically, the inner layer forming resin supplied to the first resin flow passage 62 via the first extrusion device and the outer layer forming resin supplied to the second resin flow passage 63 via the second extrusion device are formed into a cylindrical shape by the inner layer resin flow passage 63a and outer layer resin flow passage 63a, and then merged by the merge part 65, and the inner and outer layers are formed into a cylindrical shape. The cross-sectional area ratio of the inner layer and outer layer in the axial cross-section of the tube is determined by the ratio of resin merged by the merge part 65. By adjusting the supply quantity of the inner layer forming resin and the supply quantity of the outer layer forming resin and continuously varying the cross-sectional area ratio of the inner layer and outer layer in the axial cross-section, a tube containing a transition part having a prescribed length is molded. Finally, the merged resin becomes cylindrical in shape and is extruded from the opening 72a.

For example, if the inner layer forming resin supplied from the first resin flow passage 62 is hard resin and the outer layer forming resin supplied from the second resin flow passage 63 is soft resin, a tube having a hard-soft transition part can be molded. Also, if the inner layer forming resin supplied from the first resin flow passage 62 is resin of a certain color and the outer layer forming resin supplied from the second resin flow passage 63 is resin of a different color, a tube having a color transition part can be molded. Note that the inner layer forming resin and the outer layer forming resin are preferably the same type of resin, but different resins may be used provided that they are miscible.

Generally speaking, the first extrusion device and second extrusion device adjust the supply quantities of resin materials by controlling a screw or valve. However, when the supply quantity of resin material is changed, even if the supply quantity of one of the resin materials is decreased, pressure applied to the resin material beforehand ends up remaining as residual pressure, for example, inside the first resin flow passage 62 (including the inner layer resin flow passage 62a), inside the second resin flow passage 63 (including the outer layer resin flow passage 63a) and inside the merged resin flow passage 65a, and some of the resin material flows toward the land part 53 as is. This residual pressure greatly affects the dimensional stability of the tube molded while varying the cross-sectional area ratio of the inner layer and outer layer in the axial cross-section. That is, due to the effect of residual pressure, the change in cross-sectional area ratio of the inner layer and outer layer in the axial cross-section cannot be executed in a short time, and the transition part of the tube ends up being relatively long. Therefore, if this residual pressure is not suppressed, dimensional stability cannot be maintained, and the transition part of the tube cannot be shortened.

Also, in the extrusion molding die 50, the second inner layer mandrel 54 is configured such that it can move in the axial direction. For example, when the supply quantity of inner layer forming resin is decreased, residual pressure can be reduced if the flow passage volume of the inner layer resin flow passage 62a is enlarged by moving the second inner layer mandrel 54 toward the rear. On the other hand, if residual pressure is decreased, the second inner layer mandrel 54 can be moved toward the front (original state). In this way, the extrusion molding die 50 can control pressure inside the inner layer resin flow passage 62a and can shorten the distance of the land part 53 - that is, since the volume of the merged resin flow passage 65a has been reduced, the transition part of the tube can be shortened while maintaining dimensional stability. Note that the supply quantity of inner layer forming resin was described in the example, but it is also acceptable to move the second inner layer mandrel 54 in the axial direction in accordance with a change in supply quantity of the outer layer forming resin, or to respond to changes in both supply quantities. When one resin supply quantity is decreased, the other resin supply quantity is increased, and this is done to respond to residual pressure which is thought to normally occur.

Therefore, according to the extrusion molding die 50, a tube with a short transition part can be molded because changing of the supply of resin materials is smooth and the cross-sectional area ratio of the inner layer and outer layer in the axial cross-section is changed in a short time. According to the extrusion molding die 50, the length of the transition part can be selected in accordance with the application of the tube, such that it can conform to the various requirements for the tube. That is, the distance of the merged resin flow passage 65a (increase or decrease in volume of the merged resin flow passage 65a) can be determined in accordance with the application of the tube.

As above, the extrusion molding die 50 according to embodiment 1 of the present invention can suppress the effects of residual pressure remaining inside the resin flow passages (particularly the inner layer resin flow passage 62a) because it enables movement of the second inner layer mandrel 54 in the axial direction and can enlarge or shrink the flow passage volume of the inner layer resin flow passage 62a. Thus, according to the extrusion molding die 50, it is possible to shorten the distance of the merged resin flow passage 65a - that is, to reduce the volume of the merged resin flow passage 65a - while maintaining dimensional stability, and additionally, it is possible to shorten the transition part of the tube.

### Embodiment 2

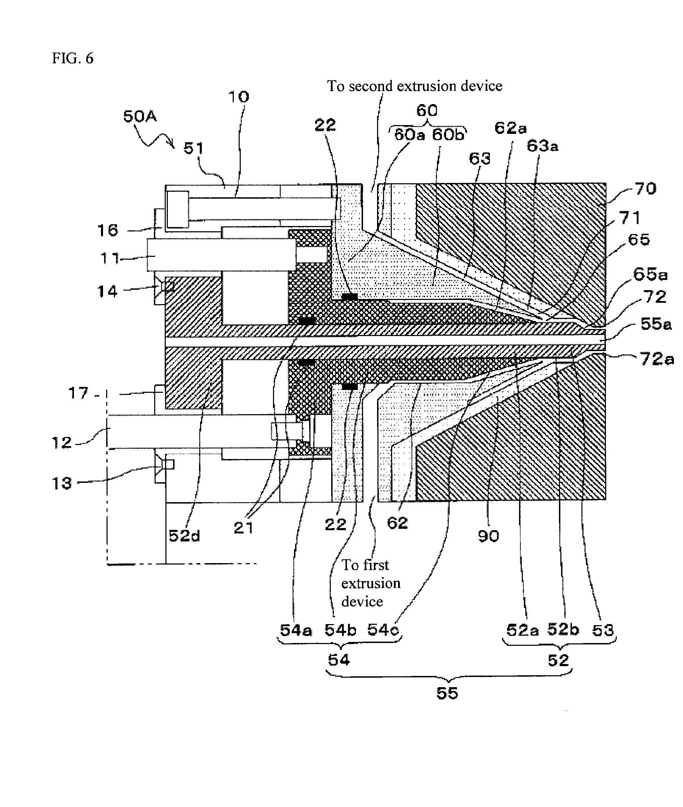

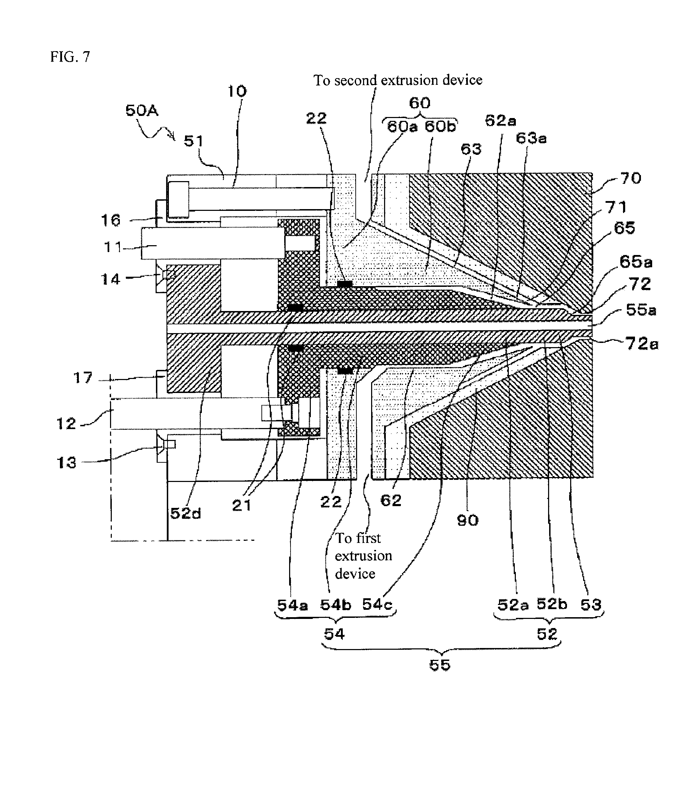

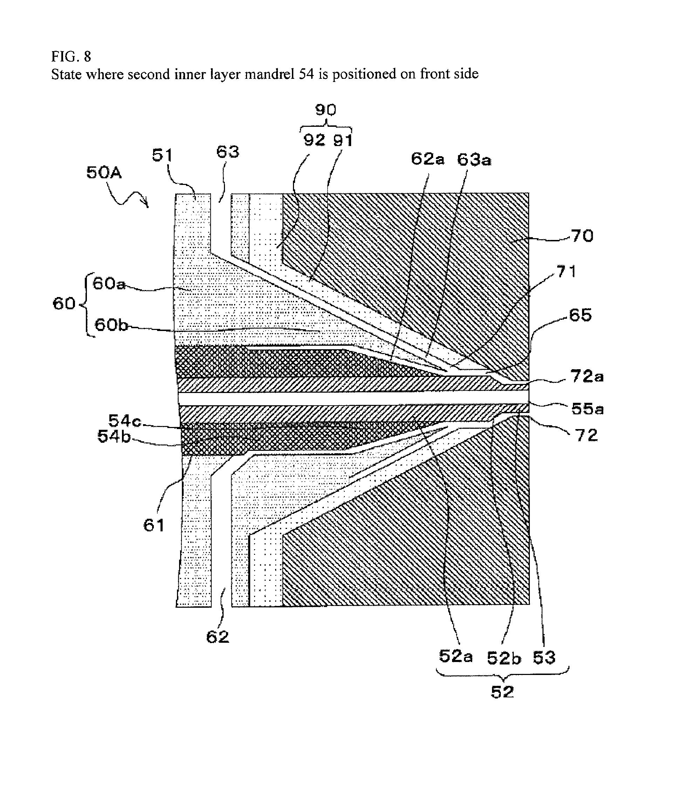

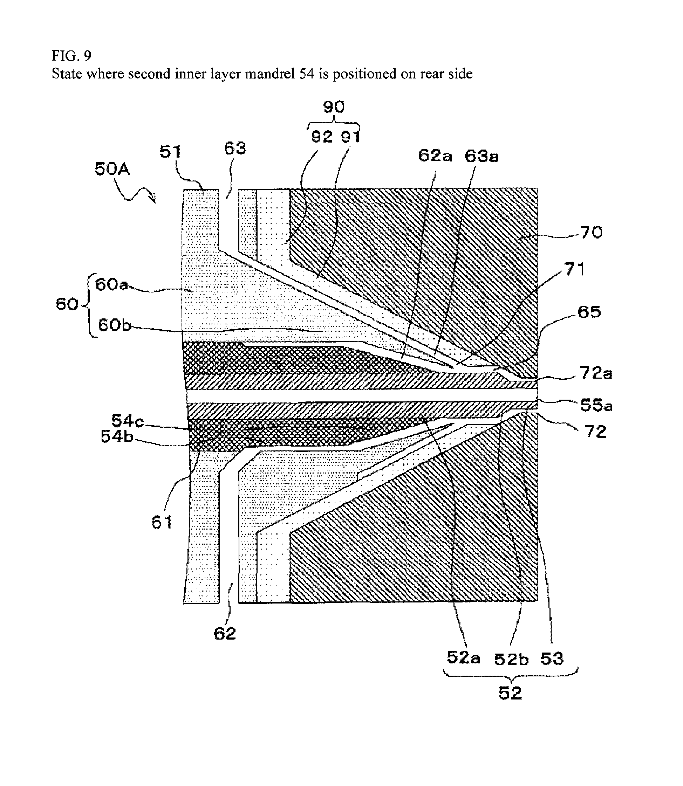

FIG. 6 and FIG. 7 are schematic cross-sectional views illustrating the cross-sectional structure of an extrusion molding die 50A according to embodiment 2 of the present invention. FIG. 8 and FIG. 9 are enlarged schematic cross-sectional views illustrating enlarged cross-sections of the main parts of the extrusion molding die 50A. The configuration and operation of the extrusion molding die 50A will be described based on FIGS. 6-9. FIGS. 6 and 8 show the state where the second inner layer mandrel 54 is positioned on the front side, and FIGS. 7 and 9 show the state where the second inner layer mandrel 54 is positioned on the rear side. Note that embodiment 2 is described while focusing on the differences from embodiment 1, and the parts that are the same as embodiment 1 are assigned the same reference numerals, and their description is omitted.

The extrusion molding die 50A according to embodiment 2 differs from the extrusion molding die 50 according to embodiment 1 in that it has a merge part adjusting collar 90. The merge part adjusting collar 90 enables adjustable setting of the distance from the merge part 65 to the tip end of the land part 53 (opening 72a) - that is, it enables adjustable setting of the distance of the merged resin flow passage 65a in advance to the desired distance. That is, by providing the merge part adjusting collar 90, the merge part 65 can be set to any position. If the position of the merge part 65 can be determined, it is also possible to determine the distance of the merged resin flow passage 65a and to further maintain the dimensional stability of the tube.

The merge part adjusting collar 90 has a tapered part 91 and a support part 92. The tapered part 91 is configured so as to have a taper angle that depends on the outer circumferential surface of the tip side tapered part 60b of the outer layer mandrel 60 and the inner circumferential surface of the tapered hole 71 of the body 70, and its inner circumferential surface forms a part of the outer layer resin flow passage 63a. One end of the support part 92 is connected to the rear side of the tapered part 91, and the other end constitutes the outer circumferential surface of the extrusion molding die 50A together with the outer layer mandrel 60 and body 70. Also, the merge part adjusting collar 90 is affixed by the support part 92 being arranged between the outer layer mandrel 60 and body 70.

Note that the shape and size, etc., of the merge part adjusting collar 90 are not limited to the shapes and sizes, etc., shown in FIGS. 6-9, and they may have any configuration so as to determine the position of the merge part 65. Also, the constituent material of the merge part adjusting collar 90 is not particularly limited, and may be a heat-resistant material such as tempered steel, for example.

Therefore, according to the extrusion molding die 50A, a tube with a short transition part can be molded because changing of the supply of resin materials is smooth and the cross-sectional area ratio of the inner layer and outer layer in the axial cross-section is changed in a short time. According to the extrusion molding die 50A, the length of the transition part can be selected in accordance with the application of the tube, such that it can conform to the various requirements for the tube. In addition, according to the extrusion molding die 50A, the distance of the land part 53 can be selected as appropriate because a merge part adjusting collar 90 is provided, which additionally contributes to dimensional stability.

As above, the extrusion molding die 50A according to embodiment 2 of the present invention can suppress the effects of residual pressure remaining inside the resin flow passages (particularly the inner layer resin flow passage 62a) because it enables movement of the second inner layer mandrel 54 in the axial direction and can enlarge or shrink the flow passage volume of the inner layer resin flow passage 62a. Also, because a merge part adjusting collar 90 is provided, the extrusion molding die 50A allows setting of the distance of the merged resin flow passage 65a to the optimal length in accordance with the tube. Thus, according to the extrusion molding die 50A, it is possible to shorten the distance of the merged resin flow passage 65a while maintaining dimensional stability, and additionally, it is possible to shorten the transition part of the tube.

### Embodiment 3

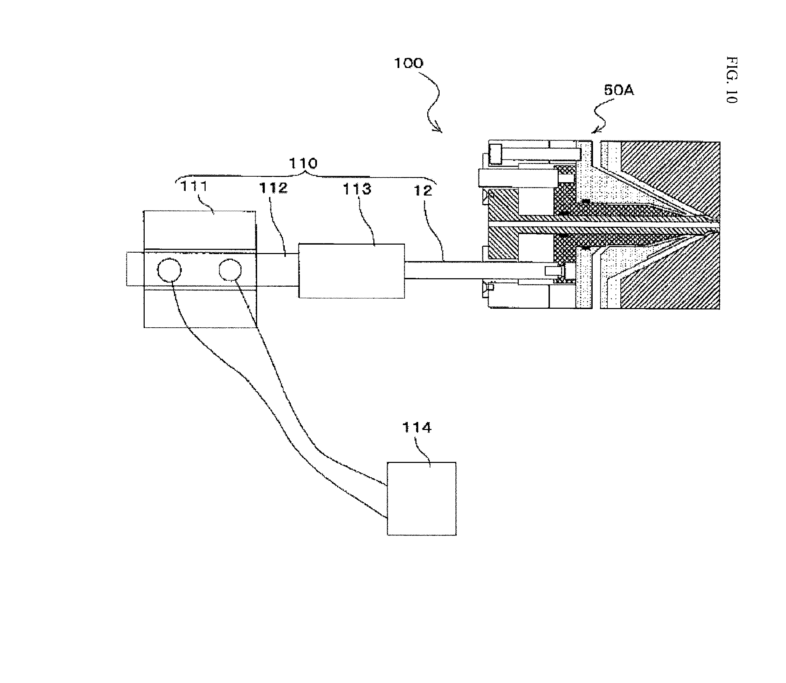

FIG. 10 is a schematic view illustrating the cross-sectional structure of an extrusion molding apparatus 100 according to embodiment 3 of the present invention. The configuration and operation of the extrusion molding apparatus 100 will be described based on FIG. 10. Note that in embodiment 3, the state where the extrusion molding apparatus 100 is equipped with the extrusion molding die 50A described in embodiment 2 is shown in the example. Thus, detailed descriptions of the extrusion molding die 50A will be omitted.

The extrusion molding apparatus 100 according to embodiment 3 is schematically constructed of the extrusion molding die 50A and a mandrel drive part 110. The extrusion molding die 50A is as described above. The mandrel drive part 110 moves the second inner layer mandrel 54 of the extrusion molding die 50A in the axial direction via a drive shaft 12. The mandrel drive part 110 has a cylinder 111, drive axis 112, coupling part 113, drive shaft 12 and controller 114. Note that even if there is no cylinder 111, it is not particularly limited provided that the second inner layer mandrel 54 can be driven; a servo motor, etc., can be used instead of the cylinder 111.

The cylinder 111 drives the drive axis 112 based on commands from the controller 114. The drive axis 112 is arranged in the cylinder 111, and is driven by the driving force produced by the cylinder 111. The coupling part 113 additionally transmits the driving force transmitted via the drive axis 112 to the drive shaft 12. The drive shaft 12, as described above, additionally transmits the driving force transmitted from the coupling part 113 to the second inner layer mandrel 54. The controller 114 controls the cylinder 111 and adjusts the driving force for driving the second inner layer mandrel 54.

The operation of the extrusion molding apparatus 100 configured as above will now be described.

The controller 114 of the extrusion molding apparatus 100 controls the cylinder 111, drives the drive axis 112 and moves the second inner layer mandrel 54 in the axial direction based on signals sent from, for example, a drawing machine (not shown in drawings). Note that the drawing machine is provided in any position which extends in the axial direction of the first inner layer mandrel 52 of the extrusion molding die 50A toward the front side, and it continuously draws the tube extruded from the extrusion molding die 50A. Also, the controller 114 of the extrusion molding apparatus 100 may also be connected to a first extrusion device and second extrusion device (not shown in drawings), so as to control the cylinder 111 by control and timing of at least one among the rotational speed of the screw, the degree of openness of the valve or the rotational speed of the gear pump provided in the first extrusion device and second extrusion device.

That is, the extrusion molding apparatus 100 moves the second inner layer mandrel 54 in accordance with changes in the supply quantities of resin materials from the first extrusion device and second extrusion device. For example, in the case where residual pressure arises in the inner layer resin flow passage 62a, etc., which is the case when the supply quantity of inner layer forming resin supplied from the first extrusion device is reduced, the extrusion molding apparatus 100 moves the second inner layer mandrel 54 toward the rear side linked with (synchronized with) the operation of the first extrusion device, and enlarges the flow passage volume of the inner layer resin flow passage 62a. Also, to reduce the residual pressure inside the inner layer resin flow passage 62a, etc., the extrusion molding apparatus 100 moves the second inner layer mandrel 54 toward the front side (original state) or leaves the second inner layer mandrel 54 as is in the current position.

In this way, because the extrusion molding apparatus 100 is equipped with an extrusion molding die 50A and a mandrel drive part 110, it can move the second inner layer mandrel 54 of the extrusion molding die 50A in the axial direction without a complex structure. Therefore, according to the extrusion molding apparatus 100, since the pressure inside the inner layer resin flow passage 62a of the extrusion molding die 50A can be adjusted as appropriate, not only can dimension stability of the tube molded by the extrusion molding die 50A be maintained, but the length of the transition part can be selected in accordance with the application of the tube, and it is possible to conform to the various requirements for the tube. Note that the pressure inside the inner layer resin flow passage 62a can be more precisely adjusted because the extrusion molding apparatus 100 is linked to the operation of its peripheral devices (for example, the first extrusion device, second extrusion device, drawing machine, cutting machine, etc.).

Note that the state where the extrusion molding apparatus 100 is equipped with the extrusion molding die 50A according to embodiment 2 was described in the example, but needless to say, the extrusion molding apparatus 100 may be equipped with the extrusion molding die 50 according to embodiment 1. Also, the mandrel drive part 110 is not limited to the configuration shown in the drawings. That is, the mandrel drive part 110 may be configured in any way provided that it can move the second inner layer mandrel 54 of the extrusion molding die 50A in the axial direction. Also, the operation of the extrusion molding apparatus 100 was described based on an example in which the cylinder 111 is controlled by timing with respect to the drawing machine, the first extrusion device and the second extrusion device, but it may also move the second inner layer mandrel 54 with any other timing, or may move it independently.

### Embodiment 4

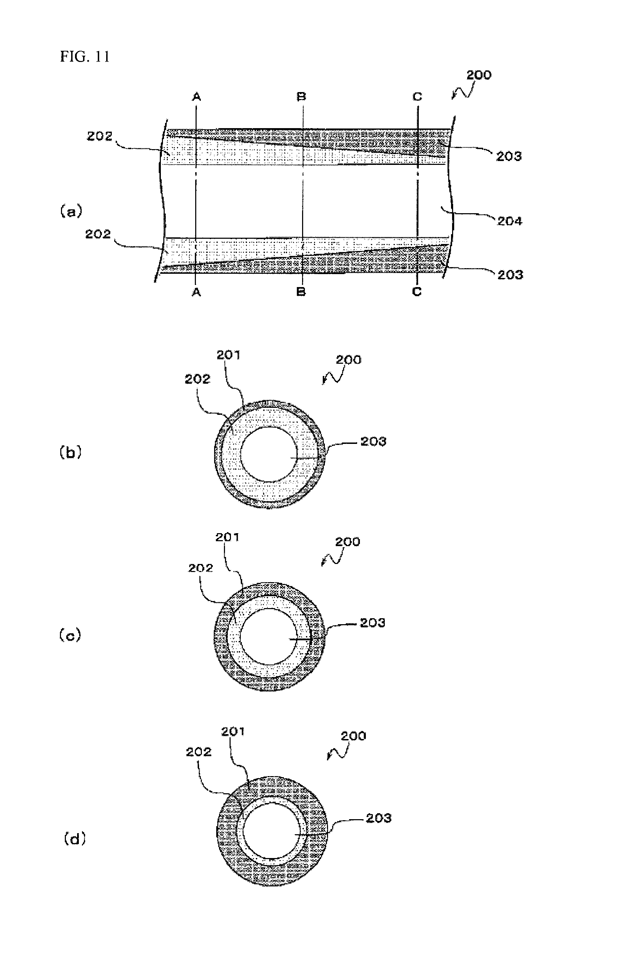

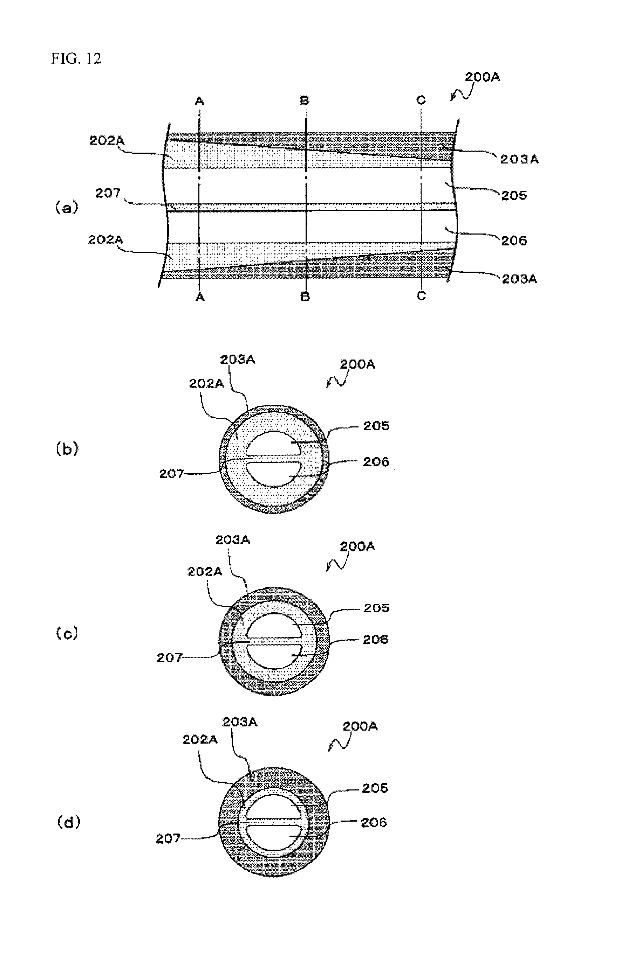

FIG. 11 is an explanatory diagram for explaining a medical tube 200 according to embodiment 4 of the present invention. FIG. 12 is an explanatory diagram for explaining the medical tube 200 of another configuration according to embodiment 4 of the present invention (for convenience of explanation, called "medical tube 200A" hereinafter). The medical tube 200 and medical tube 200A will be described based on FIG. 11 and FIG. 12. Note that the medical tube 200 and medical tube 200A according to embodiment 4 are those that were molded using the extrusion molding apparatus 100 according to embodiment 3 using the extrusion molding die 50 according to embodiment 1 or the extrusion molding die 50A according to embodiment 2. Also, in FIG. 11, the medical tube 200 is illustrated as a single lumen tube, and in FIG. 12, the medical tube 200A is illustrated as a double-lumen tube.

FIG. 11A shows the cross-sectional configuration in the axial direction of the medical tube 200, FIG. 11B shows the cross-section along line A-A shown in FIG. 11A, FIG. 11C shows the cross-section along line B-B shown in FIG. 11A, and FIG. 11D shows the cross-section along line C-C shown in FIG. 11A. Similarly, FIG. 12A shows the cross-sectional configuration in the axial direction of the medical tube 200A, FIG. 12B shows the cross-section along line A-A shown in FIG. 12A, FIG. 12C shows the cross-section along line B-B shown in FIG. 12A, and FIG. 12D shows the cross-section along line C-C shown in FIG. 12A.

The medical tube 200 according to embodiment 4 is schematically constructed of an outer layer 203 formed in a substantially cylindrical shape, and a substantially cylindrical inner layer 202 whose outer circumferential surface contacts the inner circumferential surface of the outer layer 203. Also, in the medical tube 200, a lumen 204 is formed, surrounded by the inner circumferential surface of the inner layer 202. The outer layer 203 is constructed of the outer layer forming resin supplied from the second resin flow passage 63 described above. The inner layer 202 is constructed of the inner layer forming resin supplied from the first resin flow passage 62 described above. The lumen 204 is assured to have the prescribed diameter by the land part 53 of the first inner layer mandrel 52 described above, and is formed when the outer layer 203 and inner layer 202 are extruded, in a state where pressure has been adjusted by the internal pressure adjusting hole 55a.

In the medical tube 200, the cross-sectional area ratio of the inner layer and outer layer in the axial cross-section vary continuously as shown in FIG. 11A. FIG. 11B shows the cross-sectional area ratio of the inner layer and outer layer in the axial cross-section formed in the case where the supply quantity of outer layer forming resin is smaller than the supply quantity of inner layer forming resin. FIG. 11C shows the cross-sectional area ratio of the inner layer and outer layer in the axial cross-section formed in the case where the supply quantity of outer layer forming resin and the supply quantity of inner layer forming resin are substantially the same. FIG. 11D shows the cross-sectional area ratio of the inner layer and outer layer in the axial cross-section formed in the case where the supply quantity of outer layer forming resin is greater than the supply quantity of inner layer forming resin. Note that as long as it has a transition part which contains a portion in which the cross-sectional area ratio of resin of the inner layer and outer layer in the axial cross-section continuously varies, the medical tube 200 may also contain a layer formed by only inner layer forming resin or only outer layer forming resin.

The single-lumen tube shown in FIG. 11 may be molded using a core wire. However, the structure of the extrusion molding die must be extremely complex if a core wire is to be used when molding tubes having a heteromorphous cross-section typified by a double-lumen tube (refer to FIG. 12) or a triple-lumen tube. In contrast, according to the extrusion molding die 50 according to embodiment 1 or the extrusion molding die 50A according to embodiment 2, the structure is not complex because a core wire is not used.

The medical tube 200A according to embodiment 4 is schematically constructed of an outer layer 203A formed in a substantially cylindrical shape, and a substantially cylindrical inner layer 202A whose outer circumferential surface contacts the inner circumferential surface of the outer layer 203. Also, in the medical tube 200A, the inner cavity is divided into two lumens (first lumen 205 shown on the top side of the page, second lumen 206 shown on the bottom side of the page) by a barrier wall 207 formed by inner layer forming resin, each of which is surrounded by the inner circumferential surface of the inner layer 202A and the surface of the barrier wall 207. The outer layer 203A is constructed of the outer layer forming resin supplied from the second resin flow passage 63 described above. The inner layer 202A is constructed of the inner layer forming resin supplied from the first resin flow passage 62 described above. The first lumen 205 and the second lumen 206 are assured to have the prescribed diameter by the land part 53 of the first inner layer mandrel 52 described above, and are formed when the outer layer 203A and inner layer 202A are extruded, in a state where pressure has been adjusted by the internal pressure adjusting hole 55a.

In the medical tube 200A, the cross-sectional area ratio of the inner layer and outer layer in the axial cross-section vary continuously as shown in FIG. 12A. FIG. 12B shows the cross-sectional area ratio of the inner layer and outer layer in the axial cross-section formed in the case where the supply quantity of outer layer forming resin is smaller than the supply quantity of inner layer forming resin. FIG. 12C shows the cross-sectional area ratio of the inner layer and outer layer in the axial cross-section formed in the case where the supply quantity of outer layer forming resin and the supply quantity of inner layer forming resin are substantially the same. FIG. 12D shows the cross-sectional area ratio of the inner layer and outer layer in the axial cross-section formed in the case where the supply quantity of outer layer forming resin is greater than the supply quantity of inner layer forming resin. Note that as long as it has a transition part which contains a portion in which the cross-sectional area ratio of resin of the inner layer and outer layer in the axial cross-section continuously varies, the medical tube 200A may also contain a layer formed by only inner layer forming resin or only outer layer forming resin.

If the double-lumen tube shown in FIG. 12 is molded using a core wire, the structure of the extrusion molding die ends up being extremely complex. In contrast, according to the extrusion molding die 50 according to embodiment 1 or the extrusion molding die 50A according to embodiment 2, the structure does not become complex compared to when a core wire is used, although the shape, etc., of the land part 53 must be changed. Therefore, if the extrusion molding die 50 according to embodiment 1 or the extrusion molding die 50A according to embodiment 2 is used, tubes having a heteromorphous structure as in the medical tube 200A can be molded with high efficiency.

Also, if the extrusion molding die 50 according to embodiment 1 or the extrusion molding die 50A according to embodiment 2 is used, the length of the transition part may be selected within a wide range. Therefore, if the extrusion molding die 50 according to embodiment 1 or the extrusion molding die 50A according to embodiment 2 is used, medical tubes 200 that conform to various requirements can be molded without the die itself being complex. That is, FIG. 11 shows a single-lumen tube as an example, but medical tubes having a heteromorphous structure can be molded with high efficiency provided that the extrusion molding die 50 according to embodiment 1 or the extrusion molding die 50A according to embodiment 2 is used.

Furthermore, provided that the extrusion molding die 50 according to embodiment 1 or the extrusion molding die 50A according to embodiment 2 is used, the transition part (for example, a hard-soft transition part) in which the cross-sectional area ratio of the inner layer and outer layer in the axial cross-section continuously varies can be shortened, and the applications of the medical tube 200 can be expanded.

### Embodiment 5

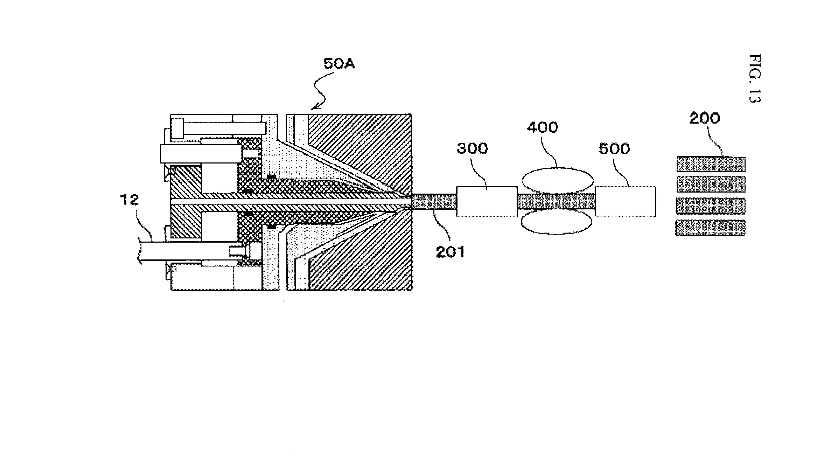

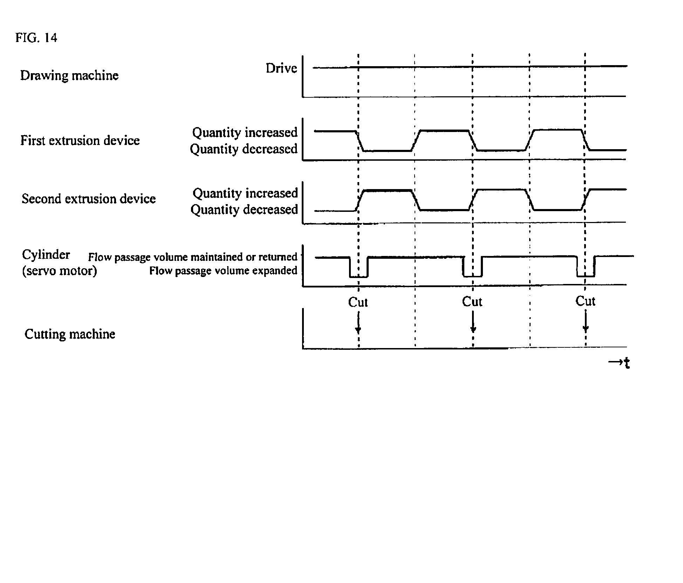

FIG. 13 is an explanatory diagram for explaining the method for producing a medical tube according to embodiment 5 of the present invention. FIG. 14 is a timing chart illustrating the operation timing of each device in the method for producing a medical tube according to embodiment 5 of the present invention. The method for producing the medical tube 200 will be described based on FIG. 13 and FIG. 14. Note that the method for producing the medical tube according to embodiment 5 is carried out using the extrusion molding apparatus 100 according to embodiment 3 which uses the extrusion molding die 50 according to embodiment 1 or the extrusion molding die 50A according to embodiment 2. FIG. 13 shows the case where the extrusion molding die 50A is used. Additionally, the horizontal axis in FIG. 14 indicates time (t), and the vertical axis indicates the state of each device.

As shown in FIG. 13, the medical tube 200 is produced by means of, from the upstream side, the extrusion molding die 50A, a tank 300, a drawing machine 400 and a cutting machine 500. In the extrusion molding die 50A, the inner layer forming resin supplied from the first extrusion device and the outer layer forming resin supplied from the second extrusion device are merged, and a tube 201 is molded. This tube 201 is extruded from the extrusion molding die 50A, and drawn by the drawing machine 400. While being drawn by the drawing machine 400, the tube 201 is cooled by cooling water held in the tank 300. After that, the tube 201 is cut to the prescribed length by the cutting machine 500 downstream from the drawing machine 400.

As shown in FIG. 14, during the production of the medical tube 200, the drawing machine 400 is in the running state. The operation of the first extrusion device and second extrusion device is controlled while their mutual timing is calculated. Also, the inner layer forming resin and outer layer forming resin are supplied from the first extrusion device and second extrusion device in quantities corresponding to the cross-sectional area ratio of the inner layer and outer layer in the axial cross-section. If the supply quantity of the inner layer forming resin supplied from the first extrusion device is decreased from the current supply quantity, residual pressure occurs in the inner layer resin flow passage 62a, etc. Thus, the controller 114 controls the cylinder 111, and moves the second inner layer mandrel 54 toward the rear side.

Since the flow passage volume of the inner layer resin flow passage 62a is enlarged when the second inner layer mandrel 54 is moved toward the rear side, residual pressure is decreased. Therefore, the cross-sectional area ratio of the inner layer and outer layer in the axial cross-section is changed in a short time. The cutting machine 500 cuts the tube 201 drawn by the drawing machine 400 to a prescribed length. For example, as shown in FIG. 14, the cutting operation of the cutting machine 500 may be synchronized with the control of the cylinder 111. If the medical tube 200 is produced with such timing, a medical tube 200 having stable dimensions can be produced with high efficiency.

Note that the method for producing a medical tube illustrated in FIG. 13 and FIG. 14 is only an example, and the method for producing a medical tube is not limited to that shown in FIG. 13 and FIG. 14 provided that it is a method for producing a medical tube that uses the extrusion molding die 50 according to embodiment 1 or the extrusion molding die 50A according to embodiment 2.

The characteristic features of the present invention were described divided into embodiments, but the present invention may be modified as appropriate within the technical scope of the present invention without limitation to the contents described by these embodiments. Also, polyurethane was cited as an example of the resin material that constitutes the medical tube, but resin materials such as, for example, polyvinyl chloride or polyamide, etc., may be used without being limited to polyurethane.

## Claims

1. An extrusion molding die (50, 50a) for extrusion-molding a tube (200, 200a) containing a portion in which a cross-sectional area ratio of an inner layer (202) and an outer layer (203) in an axial cross-section of the portion varies continuously along a length of the tube, the extrusion molding die comprising:
a main body (51);
a first inner layer mandrel (52) affixed to the main body, including a land part formed on a tip end of the first inner layer mandrel;
a second inner layer mandrel (54) having an inner circumferential surface which abuts an outer circumferential surface of the first inner layer mandrel, and which can move in an axial direction;
an outer layer mandrel (60) having a first through-hole through which the first inner layer mandrel and the second inner layer mandrel are inserted;
a body (70) having a second through-hole (72) through which the outer layer mandrel is inserted,
wherein
an outer layer resin flow passage (63a) formed from an outer circumferential surface of the outer layer mandrel and an inner circumferential surface of the second through-hole in the body and configured to enable an outer layer forming resin which forms the outer layer of the tube to flow therethrough;
an inner layer resin flow passage (62a) formed from a part of an outer circumferential surface of the second inner layer mandrel (54) and a part of the outer circumferential surface of the first inner layer mandrel (52) and an inner circumferential surface of the first through-hole in the outer layer mandrel and configured to enable an inner layer forming resin which forms the inner layer of the tube to flow therethrough; and
a merged resin flow passage (65a), in which the outer layer resin flow passage (63a) and the inner layer resin flow passage (62a) merge, formed from an outer circumferential surface of the tip end that contains the land part (53) of the first inner layer mandrel (52) and the inner circumferential surface of the second through-hole (72) in the body (70), wherein the second inner layer mandrel (54) is moved in the axial direction and the flow passage volume of the inner layer resin flow passage (62a) is changed in accordance with changes in the supply quantity of the outer layer forming resin and/or the inner layer forming resin.

2. The extrusion molding die according to claim 1, further comprising:
a merge part adjusting collar (90) positioned between the body and the outer layer mandrel configured to enable adjustment of the distance from the merge part (65), in which the outer layer resin flow passage (63a) and the inner layer resin flow passage (62a) merge, to the tip end of the land part (53).

3. An extrusion molding apparatus (100) comprising
the extrusion molding die (50) according to claim 1 or 2; and
a drive unit (110) which drives the second inner layer mandrel (54).

4. The extrusion molding apparatus according to claim 3, further comprising:
a controller (114) which controls driving of the second inner layer mandrel (54), wherein the controller (114) controls driving of the second inner layer mandrel (54) so as to enlarge the flow passage volume of the inner layer resin flow passage (62a) when the supply quantity of the outer layer forming resin and/or the inner layer forming resin is decreased.

5. A medical tube (200, 200a) extrusion-molded by the extrusion molding apparatus according to claim 3 or 4,
the medical tube comprising a transition part which contains a portion in which a cross-sectional area ratio of resin of the inner layer (202) and the outer layer (203) in the axial cross-section continuously varies along a length of the medical tube.

6. A method for producing a medical tube (200, 200a) which contains a portion in which a cross-sectional area ratio of resin of an inner layer (202) and an outer layer (203) in the axial cross-section of the portion continuously varies along a length of the medical tube, the method comprising:
providing an extrusion molding die including an outer layer resin flow passage (63a), an inner layer resin flow passage (62a) and a merged resin flow passage (65a); and
adjusting a volume of the inner layer resin flow passage (62a) to thereby adjust pressures in the outer layer resin flow passage (63a), the inner layer resin flow passage (62a) and the merged resin flow passage (65a).

7. The method of claim 6 wherein the step of adjusting a volume of the inner layer resin flow passage (62a) includes adjusting the volume of the inner layer resin flow passage (62a) in relation to changes in the supply quantity of the outer layer forming resin or the inner layer forming resin.

8. The method of claim 6 wherein the step of adjusting a volume of the inner layer resin flow passage (62a) includes adjusting the volume of the inner layer resin flow passage (62a) in relation to changes in the supply quantity of the outer layer forming resin and the inner layer forming resin.

9. The method of claim 6 wherein the step of adjusting a volume of the inner layer resin flow passage (62a) includes adjusting the volume of the inner layer resin flow passage (62a) by moving an inner layer mandrel in an axial direction.
